# EUROPEAN PATENT APPLICATION

(11) **EP 4 483 819 A1**
(43) Date of publication of application: **01.01.2025**
(21) Application number: 24168379.6
(22) Date of filing: 04.04.2024
(51) Int. Cl.: A61B 17/16, A61B 17/92, A61F 2/46

(54) **ORTHOPEDIC INSTRUMENT CONNECTION MECHANISMS AND RELATED ASSEMBLIES AND SYSTEMS**

(30) Priority: 30.06.2023 US 202363511407 P
(71) Applicant: MicroPort Orthopedics Holdings Inc., Arlington, TN 38002 (US)
(72) Inventor: Hollandsworth, Michael D., Arlington, TN 38002 (US); Bowman, Fred W., Arlington, TN 38002 (US); Brooks, Michael L., Arlington, TN 38002 (US)
(74) Representative: Novagraaf International SA

(57) **Abstract**

A medical device connection mechanism, related assemblies and systems comprising generally: a first medical device having: a protrusion, the protrusion including a proximal end and a distal end distally disposed from the proximal end along a height of a body of the protrusion, the body of the protrusion comprising an interrupted threaded portion disposed closer to the distal end than the proximal end; and a second medical device having: an interior sidewall, the interior sidewall defining a hole extending into the second medical device, a discontinuous threaded portion disposed within the hole, the discontinuous threaded portion defining a threadless channel extending along a discontinuous threaded portion height, and the discontinuous threaded portion configured to receive the interrupted threaded portion of the protrusion of the first medical device through the threadless channel.

## Description

### BACKGROUND OF THE INVENTION

### 1. Technical Field

The present disclosure relates generally to the field of orthopedic surgery, and more particularly to an orthopedic instrument connection mechanism and related systems and assemblies.

### 2. Related Art

In many minimally invasive orthopedic surgical procedures, the surgeon uses a variety of tools to prepare and orient endoprosthetic implants. Many such instruments comprise a handle and an adaptor. The adaptor can connect to a variety of broaching, reaming, and placement tools that are selectively employed throughout the procedure. To work effectively and to minimize the time that a patient is under anesthesia, the surgeon typically works with a team of technicians, nurses, and other medical professionals to assemble and prepare the various sterilized surgical instruments prior to use.

Some prior orthopedic medical device connection mechanisms consisted of single points of engagement. While these were generally quick to connect and disconnect, these designs could lead to inaccurate or lose fittings of the instruments. Other designs included delicate and complex spring and pin mechanisms, which could be prone to failure after repeated impaction.

Other prior devices, such as the one disclosed by Dees et. al. in PCT. App. No. PCT/US2016/032346, required the use of a guide rod. Guide rods can restrict alignment possibilities and the installation and adjustment of such guide rods can prolong procedure time.

### SUMMARY OF THE INVENTION

The problems of the prior art are solved by exemplary orthopedic medical device connection mechanisms in accordance with the present disclosure. An exemplary orthopedic medical device connection mechanism comprises: a first medical device having: a protrusion, the protrusion including a proximal end and a distal end distally disposed from the proximal end along a height of a protrusion body, the protrusion body comprising an interrupted threaded portion disposed closer to the distal end than the proximal end; and a second medical device having: a body, an interior sidewall, the interior sidewall defining a hole extending into the body, a discontinuous threaded portion disposed within the hole, the discontinuous threaded portion defining a threadless channel extending along a discontinuous threaded portion height, and the discontinuous threaded portion configured to receive the interrupted threaded portion of the protrusion of the first medical device through the threadless channel. The present invention is defined in the appended claims.

It is contemplated that the orthopedic medical device connection mechanism described herein may permit quick, accurate, and secure assembly and disassembly of orthopedic instruments, while permitting the connection mechanism components to survive repeated blunt force from impaction instruments.

It is further contemplated that the orthopedic medical device connection mechanism described herein may permit the use of a variety of modular orthopedic medical device assemblies that can be configured to be assembled and disassembled via an exemplary medical device connection mechanism in accordance with this disclosure.

It is still further contemplated that the orthopedic medical device connection mechanism described herein may permit the use of fewer manual orthopedic instruments at the time of surgery compared to prior designs that utilized complex connection mechanisms.

It is yet still further contemplated that the orthopedic medical device connection mechanism described herein may permit the user to use orthopedic medical devices in free form (*e*.*g*., without being restricted by guiding instrumentation such as an intramedullary rod).

It is still yet further contemplated that the orthopedic medical device connection mechanism described herein may permit the use of modular medical devices such as modular broaches, wherein a modular broach component of the modular broach is able to be used with any other modular broach component in a provided supply of such components to thereby better accommodate each patient's unique anatomy while minimizing inventory.

### BRIEF DESCRIPTION OF THE DRAWINGS

The foregoing will be apparent from the following more particular description of exemplary embodiments of the disclosure, as illustrated in the accompanying drawings. The drawings are not necessarily to scale, with emphasis instead being placed upon illustrating the disclosed embodiments.
**FIG. 1** is an isometric view of an exemplary first medical device, wherein the first medical device is an adaptor of an orthopedic implant assembly.
**FIG. 2** is an isometric view of an example second medical device, wherein the second medical device is an orthopedic instrument that is configured to receive the adaptor.
**FIG. 3** is a detailed perspective view of the medical device of **FIG. 2****.**
**FIG. 4** is an isometric view of an orthopedic instrument assembly having an exemplary medical device connection mechanism and positioner; the orthopedic instrument assembly is depicted in an assembled configuration.
**FIG. 5** is a close up perspective cross-sectional side view of the orthopedic instrument assembly of **FIG. 4****.**
**FIG. 6** is an isometric expanded view of an exemplary orthopedic instrument assembly having an exemplary medical device connection mechanism; the exemplary orthopedic instrument assembly comprises stacked modular orthopedic instruments, depicted in a disassembled configuration.
**FIG. 7** is an isometric view of the exemplary orthopedic instrument assembly of **FIG. 6****,** depicted in an assembled configuration.
**FIG. 8A** is a bottom view of the exemplary orthopedic instrument assembly of **FIG. 7****,** depicted in a partially assembled configuration.
**FIG. 8B** is a bottom view of the exemplary orthopedic instrument assembly of **FIG. 7****,** depicted in a fully assembled configuration.
**FIG. 9** is a facing view of an exemplary orthopedic instrument assembly in a pre-engagement configuration.
**FIG. 10** is an isometric view of an exemplary orthopedic instrument assembly in an assembled configuration, wherein the second medical device of the depicted orthopedic instrument assembly is a secondary adaptor.
**FIG. 11** is an isometric view of the orthopedic instrument assembly of **FIG. 10** further depicting an endoprosthetic implant engaged to the secondary adaptor.
**FIG. 12** is an isometric view of the orthopedic instrument assembly of **FIG. 11** engaged to a positioner, wherein the endoprosthetic implant has been inserted into a resected proximal tibia.

### DETAILED DESCRIPTION OF THE PREFERRED EMBODIMENTS

The following detailed description of the preferred embodiments is presented only for illustrative and descriptive purposes and is not intended to be exhaustive or to limit the scope and spirit of the invention. The embodiments were selected and described to best explain the principles of the invention and its practical application. One of ordinary skill in the art will recognize that many variations can be made to the invention disclosed in this specification without departing from the scope and spirit of the invention.

Similar reference characters indicate corresponding parts throughout the several views unless otherwise stated. Although the drawings represent embodiments of various features and components according to the present disclosure, the drawings are not necessarily to scale and certain features may be exaggerated to better illustrate embodiments of the present disclosure, and such exemplifications are not to be construed as limiting the scope of the present disclosure.

Except as otherwise expressly stated herein, the following rules of interpretation apply to this specification: (a) all words used herein shall be construed to be of such gender or number (singular or plural) as such circumstances require; (b) the singular terms "a," "an," and "the," as used in the specification and the appended claims include plural references unless the context clearly dictates otherwise; (c) the antecedent term "about" applied to a recited range or value denotes an approximation with the deviation in the range or values known or expected in the art from the measurements; (d) the words, "herein," "hereby," "hereto," "hereinbefore," and "hereinafter," and words of similar import, refer to this specification in its entirety and not to any particular paragraph, claim, or other subdivision, unless otherwise specified; (e) descriptive headings are for convenience only and shall not control or affect the meaning of construction of part of the specification; and (f) "or" and "any" are not exclusive and "include" and "including" are not limiting. Further, the terms, "comprising," "having," "including," and "containing" are to be construed as open-ended terms (*i.e.*, meaning "including but not limited to").

References in the specification to "one embodiment," "an embodiment," "an exemplary embodiment," *etc.*, indicate that the embodiment described may include a particular feature, structure, or characteristic, but every embodiment may not necessarily include the particular feature, structure, or characteristic. Moreover, such phrases are not necessarily referring to the same embodiment. Further, when a particular feature, structure, or characteristic is described in connection with an embodiment, it is submitted that it is within the knowledge of one skilled in the art to affect such feature, structure, or characteristic in connection with other embodiments, whether explicitly described.

To the extent necessary to provide descriptive support, the subject matter and/or text of the appended claims are incorporated herein by reference in their entirety.

Recitation of ranges of values herein are merely intended to serve as a shorthand method of referring individually to each separate value falling within the range of any sub-ranges there between, unless otherwise clearly indicated herein. Each separate value within a recited range is incorporated into the specification or claims as if each separate value were individually recited herein. Where a specific range of values is provided, it is understood that each intervening value, to the tenth or less of the unit of the lower limit between the upper and lower limit of that range and any other stated or intervening value in that stated range of sub range thereof, is included herein unless the context clearly dictates otherwise. All subranges are also included. The upper and lower limits of these smaller ranges are also included therein, subject to any specifically and expressly excluded limit in the stated range.

The terms, "horizontal" and "vertical" are used to indicate direction relative to an absolute reference, *i.e.*, ground level. However, these terms should not be construed to require structure to be absolutely parallel or absolutely perpendicular to each other. For example, a first vertical structure and a second vertical structure are not necessarily parallel to each other.

Throughout this disclosure and unless otherwise noted, various positional terms, such as "distal," "proximal," "medial," "lateral," "anterior," and "posterior," will be used in the customary manner when referring to the human anatomy. More specifically, "distal" refers to the area away from the point of attachment to the body, while "proximal" refers to the area near the point of attachment to the body. For example, the distal femur refers to the portion of the femur near the tibia, whereas the proximal femur refers to the portion of the femur near the hip. The terms, "medial" and "lateral" are also essentially opposites. "Medial" refers to something that is disposed closer to the middle of the body. "Lateral" means that something is disposed closer to the right side or the left side of the body than to the middle of the body. Regarding, "anterior" and "posterior," "anterior" refers to something disposed closer to the front of the body, whereas "posterior" refers to something disposed closer to the rear of the body."

"Varus" and "valgus" are broad terms and include without limitation, rotational movement in a medial and/or lateral direction relative to the knee j oint.

The term, "mechanical axis" of the femur refers to an imaginary line drawn from the center of the femoral head to the center of the distal femur at the knee.

The term, "anatomic axis" refers to an imaginary line drawn lengthwise down the middle of femoral shaft or tibial shaft, depending upon use.

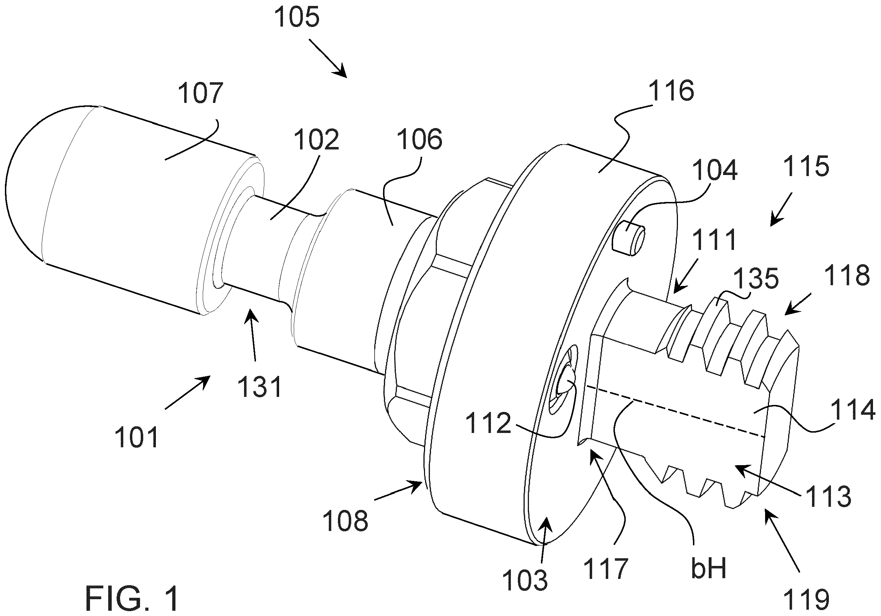

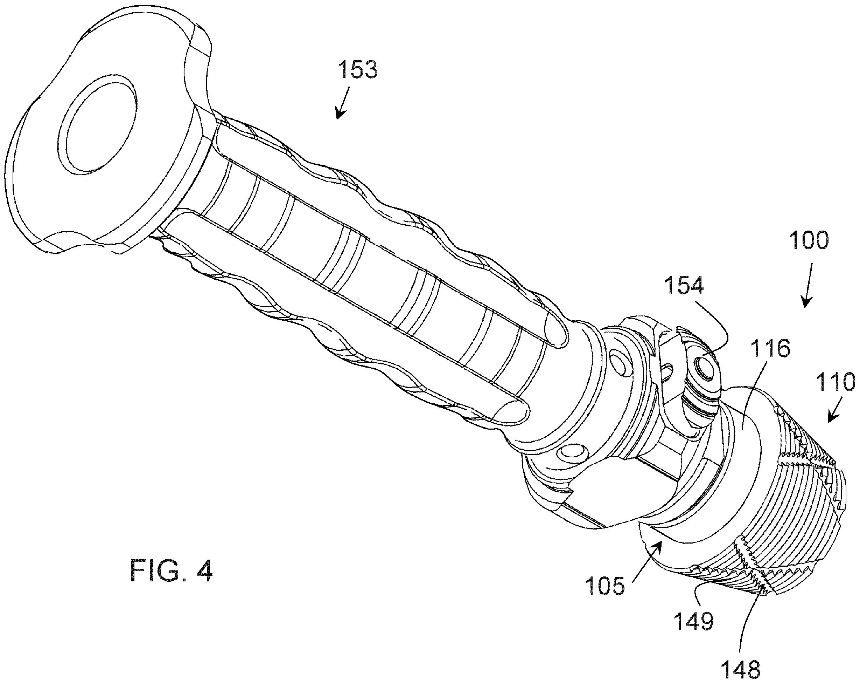

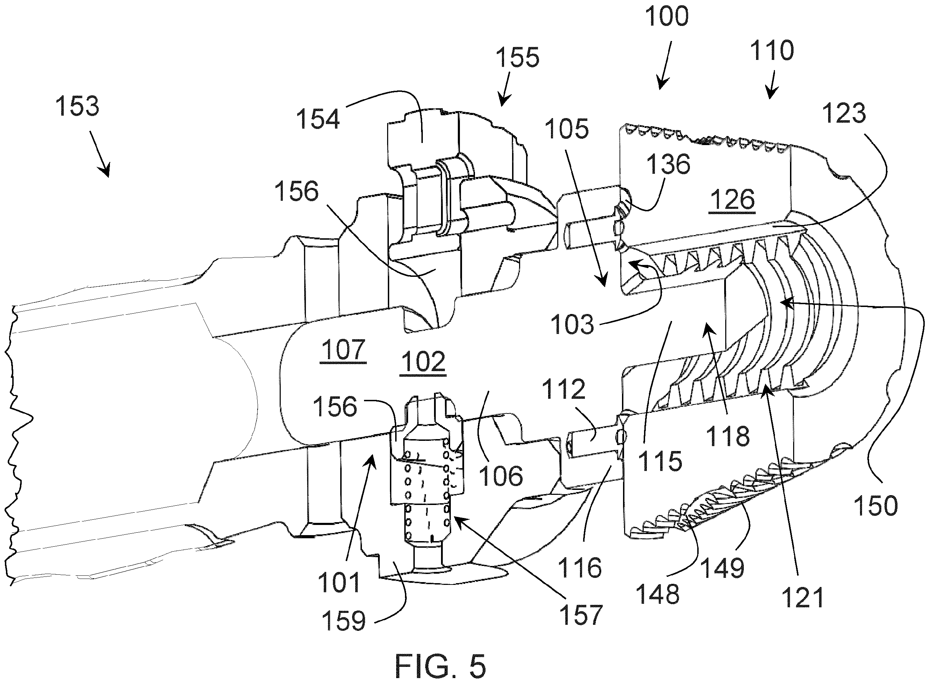

**FIG. 1** depicts an exemplary first medical device **105** of an orthopedic instrument assembly **100** (**FIG. 4**) comprising an exemplary medical device connection mechanism **150** (**FIG. 5**). It will be appreciated that the medical devices disclosed herein are generally contemplated to be used in surgical procedures, particularly in orthopedic surgical procedures. It is contemplated that in certain exemplary embodiments, the medical devices may be orthopedic instruments, orthopedic implants, orthopedic instrument adaptors, combinations or components thereof; however, nothing in this disclosure limits the contemplated "medical devices" to the examples provided. In the depicted exemplary embodiment, the first medical device **105** is an adaptor that comprises a protrusion **115** having a proximal end **117** and a distal end **119** distally disposed from the proximal end **117** along a height **bH** of a protrusion body **113,** the protrusion body **113** can optionally include a shank portion **111** disposed closer to the proximal end **117** than the distal end **119.** An interrupted threaded portion **118** is disposed closer to the distal end **119** than the proximal end **117.** In other exemplary embodiments, the non-threaded shank portion **111** can be absent and the interrupted threaded portion **118** can extend to the proximal end **117.**

The interrupted threaded portion **118** desirably comprises timed screw threads **135** that extend generally radially outward from the non-interrupted portion of the protrusion body **113.** In the depicted embodiment, the interrupted portion of the protrusion body **113** comprises two shear faces **114** disposed at opposing sides of the protrusion body **113** and that extend generally along the height **bH** of the protrusion body **113.**

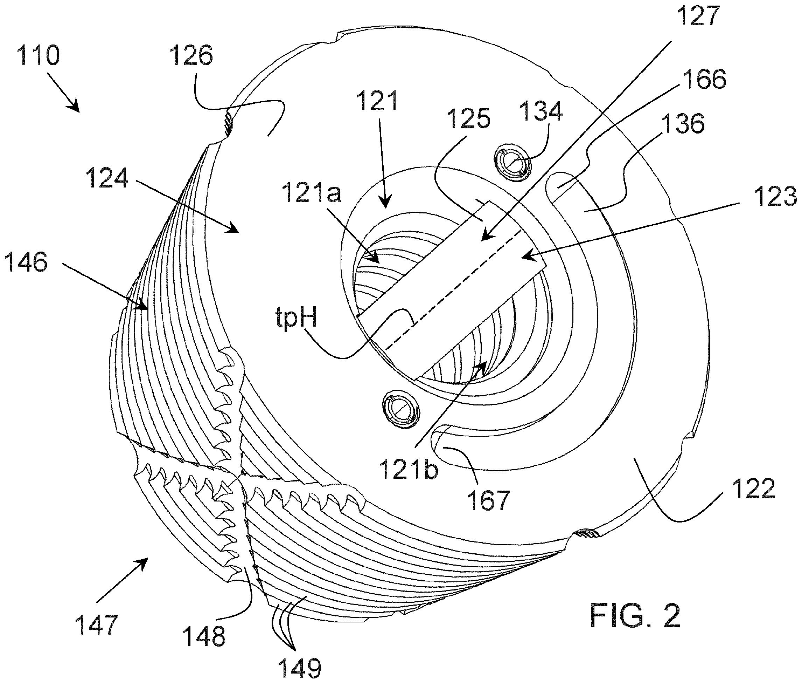

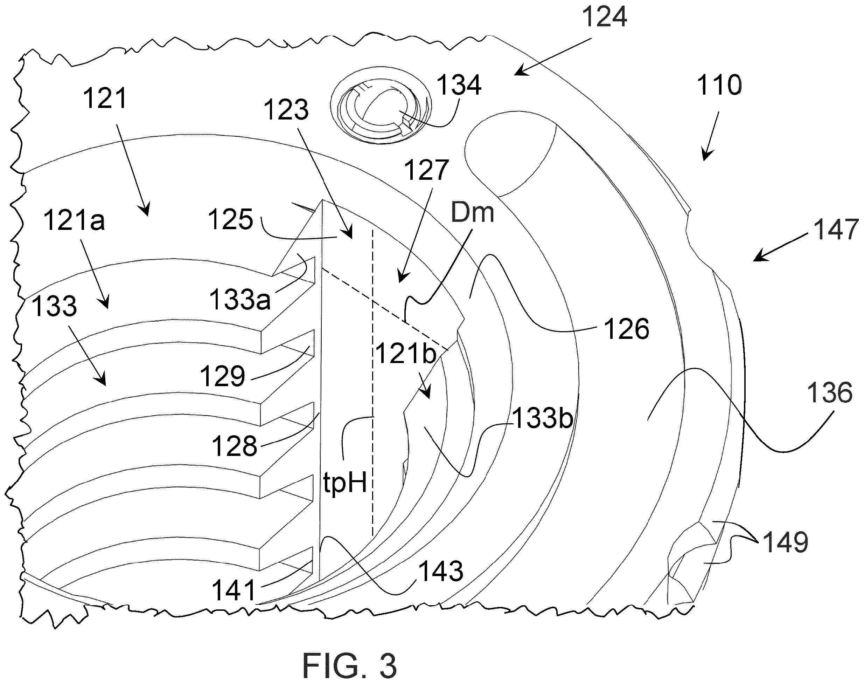

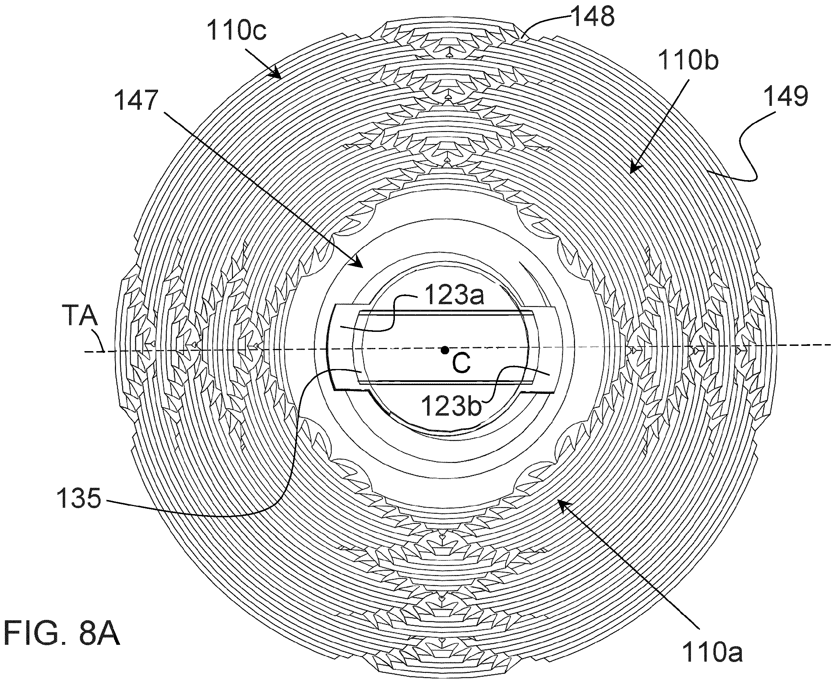

**FIG. 2** depicts an exemplary second medical device **110** of an orthopedic instrument assembly **100** comprising an exemplary medical device connection mechanism **150.** The second medical device **110** comprises a body **126** having a proximal side **124** distally disposed from a distal side **147** and an interior sidewall **129** (**FIG. 3**) extending between the proximal side **124** and the distal side **147.** The interior sidewall **129** defines a hole **127** extending into the body **126** of second medical device **110.** A discontinuous threaded portion **121** is disposed within the hole **127.** In the depicted embodiment, threads **133** (**FIG. 3**) of the discontinuous threaded portion **121** extend from the interior sidewall **129** radially inward toward a center longitudinal axis **C** (**FIG. 8A**) of the hole **127.** The discontinuous threaded portion **121** defines a threadless channel **123** extending along a discontinuous threaded portion height **tpH.** Stated differently, the absence of screw threads **133** along a portion of the discontinuous threaded portion height **tpH** defines a threadless channel **123.** The distance **D** between adjacent discontinuous threads **133a, 133b** (**FIG. 3**) on either side of the threadless channel **123** is desirably sized to closely receive the interrupted threaded portion **118** of the protrusion **115** when the first medical device **105** and the second medical device **110** begin to be engaged (*i.e.*, are disposed in a "pre-engagement" or "preassembled" configuration, see **FIG. 8A**). In this manner, the discontinuous threaded portion **121** can be said to be "configured to receive" the interrupted threaded portion **118** of the protrusion **115** of the first medical device **105** through the threadless channel **123.** Furthermore, a threadless channel **123** that can closely receive the interrupted threaded portion **118** of the protrusion **115** of the first medical device **105** can be said to be a "keyed slot" or a "keyed threadless channel" **123.**

In the depicted embodiments, the distance **D** between adjacent discontinuous threads **133a, 133b** on either side of the threadless channel **123** is substantially uniform among the multiple adjacent discontinuous threads **133a, 133b.** Without being bound by theory, it is contemplated that this arrangement is desirable because it can facilitate quick guidance of the interrupted threaded portion **118** of the protrusion **115** into the discontinuous threaded portion **121,** especially in situations with limited visibility. However, it will be appreciated that this disclosure is not limited to embodiments comprising threadless channels **123** defined by adjacent discontinuous threads **133a, 133b** disposed at a uniform distance **D.** In certain embodiments, one or more discontinuous threads that are superiorly or inferiorly disposed to a pair of adjacent reference discontinuous threads **133a, 133b** disposed on either side of the threadless channel **123** that are separated by a distance **D,** can be separated by a threadless channel distance that is greater than or lesser than the distance **D** of the reference discontinuous threads **133a, 133b.**

As better seen in **FIG. 3****,** the threadless channel **123** can be recessed into the body **126** of the second medical device **110.** Stated another way, the threadless channel interior sidewall **125** is disposed radially outward from the interior sidewall **129.** A step **128** extends radially between an edge of **141** of the interior sidewall **129** and an edge **143** of the adjacent threadless channel **123.** In this manner, the threadless channel **123** can be said to be "recessed within the body **126** of the second medical device **110."** It will be appreciated that that same step and edge structure is typically present on the other side of the threadless channel **123.** In the depicted embodiment, a second keyed slot **123b** is disposed opposite from the first keyed slot (**FIG. 2**). It will be appreciated that in other exemplary embodiments, the threadless channel interior sidewall **125** can be absent such that the threadless channel **123** further extends radially through the body **126** of the second medical device **110.** However, in certain exemplary embodiments, the discontinuous threaded portion **121** can be interrupted by a single keyed slot **123a.** In other exemplary embodiments, more than two keyed slots can comprise the discontinuous threaded portion **121.**

Without being bound by theory, it is further contemplated that a keyed slot **123** that is recessed within the body **126** of the second medical device **110** can further facilitate the alignment and guidance of the interrupted threaded portion **118** of the protrusion **115** of the first medical device **105** when connecting or disconnecting the exemplary connection mechanisms **150** disclosed herein. This is contemplated to be particularly beneficial when visibility is limited. The recessed keyed slot **123** can permit the first medical device **105** to be guided into the second medical device **110** by feel, which can contribute to efficient assembly or disassembly of orthopedic instrument assemblies **100** comprising an exemplary connection mechanism **150.**

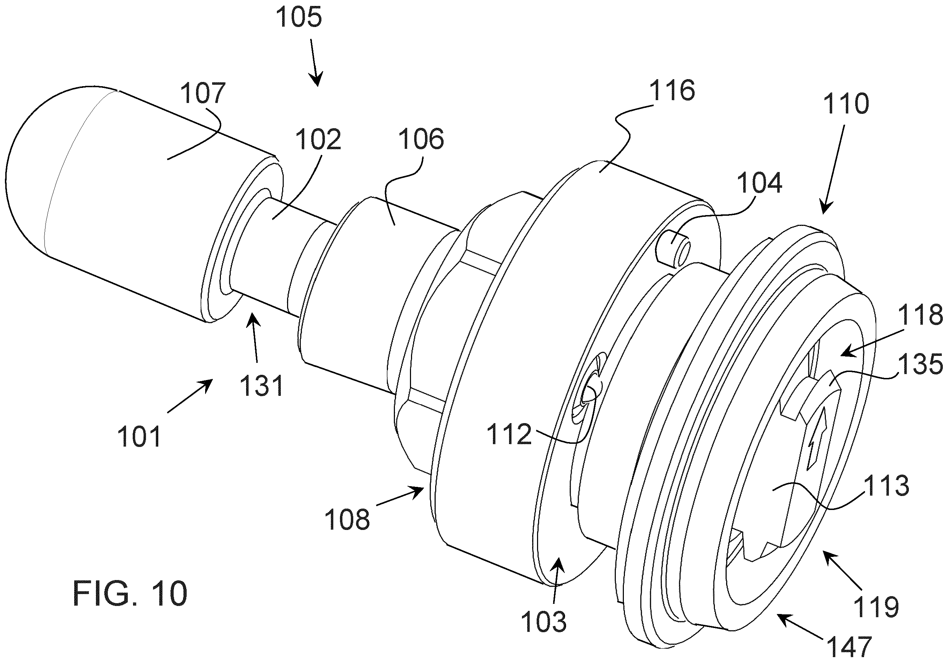

It will be appreciated that if similar edge **141, 143** and step **128** structures are present in an orthopedic implant, the keyed slot **123** can be said to be "recessed within the body of the orthopedic implant." Similarly, it will be appreciated that if similar edge **141, 143** and step **128** structures are present on a secondary adaptor (see **FIG. 10**), the keyed slot **123** can be said to be "recessed within the body of the secondary adaptor." In any such embodiment, the opposing steps **128** and the threadless channel interior sidewall **125** can be said to further define the keyed slot **123.** The depicted exemplary embodiments comprise two opposing threadless channels **123.** As such, the depicted discontinuous threaded portion **121** can be said to comprise a first portion of discontinuous threads **121a** that is opposed to a second portion of discontinuous threads **121b.** However, it will be appreciated that in other exemplary embodiments, a single threadless **123** may be present. In yet other exemplary embodiments, more than two threadless channels **123** can be present. In embodiments having multiple threadless channels **123,** the threadless channels are desirably disposed at regular intervals relative to the adjacent threadless channel **123** or threadless channels **123.** However, it will be appreciated that in other exemplary embodiments, the multiple threadless channels **123** can be disposed at irregular intervals relative to the adjacent threadless channel **123** or threadless channels **123.**

In exemplary embodiments, the threads **133** of the discontinuous threaded portion **121** and the threads **135** of the complementary interrupted threaded portion **118** of the protrusion **115** desirably have a trapezoidal cross-section (see **FIG. 3**) when bisected radially. Without being bound by theory, it is contemplated that screw threads **133, 135** having a trapezoidal cross-section, such as an ACME thread form or a trapezoidal metric thread form, can facilitate increased engagement speed compared to standard threads in part by generally having a lower number of threads per axial distance, having additional strength at the base of the threaded form, and having increased thread shear capacity. It is further contemplated that such trapezoidal threads **133,135,** when in cam locking engagement with each other may effectively transfer impaction forces more uniformly through the engaged threads **133, 135** and thereby survive repeated impaction longer compared to non-trapezoidal screw threads. However, nothing in this disclosure limits the screw threads to ACME, trapezoidal metric, or otherwise trapezoidal screw threads. All types of screw threads are considered to be within the scope of this disclosure, including but not limited to standard , V-shaped, American National, Unified, metric, square, ACME, British (Whitworth Standard), knuckle, and buttress type screw threads.

Without being bound by theory, it is contemplated that the threads **133** of the discontinuous threaded portion **121** and the threads **135** of the interrupted threaded portion **118** can desirably be "timed" or "clocked" such that placement of a given orthopedic instrument assembly **100** into the assembled configuration results in a known angular displacement and a known travel depth of the protrusion **115** relative to a starting position of the protrusion **115** (*i.e.*, in the preassembled configuration).

The "thread timing" or "thread clocking" is a function of the pitch and spacing of the thread. In practice, when the threads **135, 133** are placed in the preassembled configuration (see **FIG. 8A**), the threads **135** of interrupted threaded portion **118** are placed in the threadless channels **123a, 123b** of the discontinuous threaded portion **121.** In the embodiment depicted in **FIG. 8A****,** a first transverse axis **TA** can be imagined to extend between the opposing threadless channels **123a, 123b** and bisect the interrupted threaded portion **118** laterally. When placed in the preassembled configuration, the interrupted threaded portion **118** and the discontinuous threaded portion **121** share a common center longitudinal axis **C.** With timed threads, the starting point of thread engagement is determined by the thread pitch relative to the shared rotational axis between the internal **135** and external **133** threads and the length of the protrusion **115.** As the orthopedic instrument assembly **100** is rotated from the preassembled configuration to the assembled configuration, the threads **135** of the interrupted threaded portion **118** begin to mesh with the threads **133** of the discontinuous threaded portion **121.** With timed threads, the length of the protrusion travels a known distance into the second medical device **110** with every angular displacement. To facilitate quick connection, it is contemplated that less than a full turn to fully mesh and engage the respective threads **135, 133** can be desirable. It is contemplated that a single-point thread mill can be used to create the threaded forms described herein.

Timed threads are thought to be particularly advantageous to the exemplary medical device connection mechanisms contemplated herein because of visibility and time constraints in the operating room. If a surgeon or technician can assemble orthopedic instrument assemblies **100** having an exemplary medical device connection mechanism **150** without looking at the assembly and know that the assembly is fully assembled and secured when the first medical device **105** is rotated a known number of degrees ("°") (*e.g.*, 90° in certain embodiments), the surgeon can continue with the procedure with minimal interruption. This can ultimately contribute to less patient time under anesthesia and improved patient safety.

Furthermore, without being bound by theory, it is contemplated that the use of trapezoidal and timed threads in exemplary embodiments, together with the interrupted threaded portion **118** and the discontinuous threaded portion **121,** can permit the respective threads **133, 135** to mesh (*i.e.*, in the assembled configuration) faster (*e.g.*, in a quarter turn) than a fully threaded connection. A fully threaded connection would require several turns to engage the respective components and could result in the components not fully threading into one another, which could undermine the stability of the connection, especially when subject to impaction forces common in orthopedic procedures.

It will be further appreciated that exemplary orthopedic medical device connection mechanisms **150** in accordance with this disclosure effectively transfer impaction forces around the meshed screw threads **133, 135,** when the orthopedic medical device connection mechanism **150** is in the assembled configuration. This feature can reduce wear of the connection mechanism **150** during use, while permitting quick assembly and disassembly of orthopedic medical devices having the mating components of the connection mechanism **150,** which can ultimately contribute to a reduction in patient time under anesthesia.

Although the protrusion **115** has been described herein as extending from the first medical device **105** and the discontinuous threaded form **121** has been described as being disposed within the hole **127** of the second medical device **110,** it will be appreciated that in other exemplary embodiments, the protrusion **115** can extend from the second medical device **110** and be configured to mate (in any manner described throughout this disclosure) with a discontinuous threaded form **121** disposed within a hole **127** of the first medical device **105.**

Although complementary threads **133, 135** are considered to be the most robust and reliable type of engagement mechanism for the exemplary medical device engagement mechanisms described herein, it will be appreciated that all other rotationally engaged mechanical engagement mechanisms are considered to be within the scope of this disclosure. Non-limiting examples of such other rotationally engaged mechanical engagement mechanisms can include one or more further protrusions extending from the first medical device **105,** which may include (by way of further example) one or more hooks, clamps, clasps, ramps, ledges, or ridges, and one or more receivers disposed in the second medical device **110,** the one or more receivers being configured to closely receive the one or more further protrusions from the first medical device **105.** The further receivers may include (by way of further example) one or more complementary rods, clamps, clasps, ramps, loops, arches, ledges, recessions, gaps, or ridges configured to closely receive the one or more further protrusions from the first medical device **105.** It will be appreciated that in other exemplary embodiments, the receiver(s) can be on the first medical device **105** and the further protrusions can be on the second medical device **110.** Combinations and permutations of the forgoing are considered to be within the scope of this disclosure.

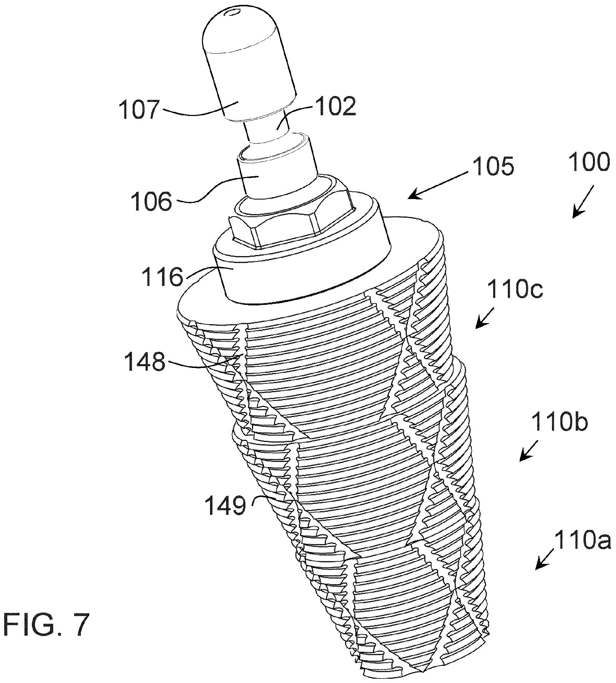

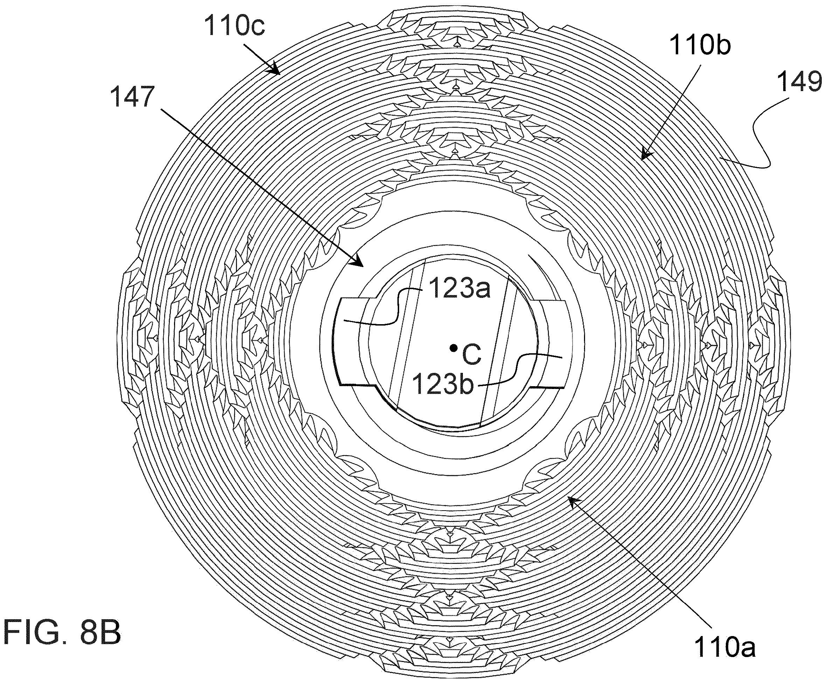

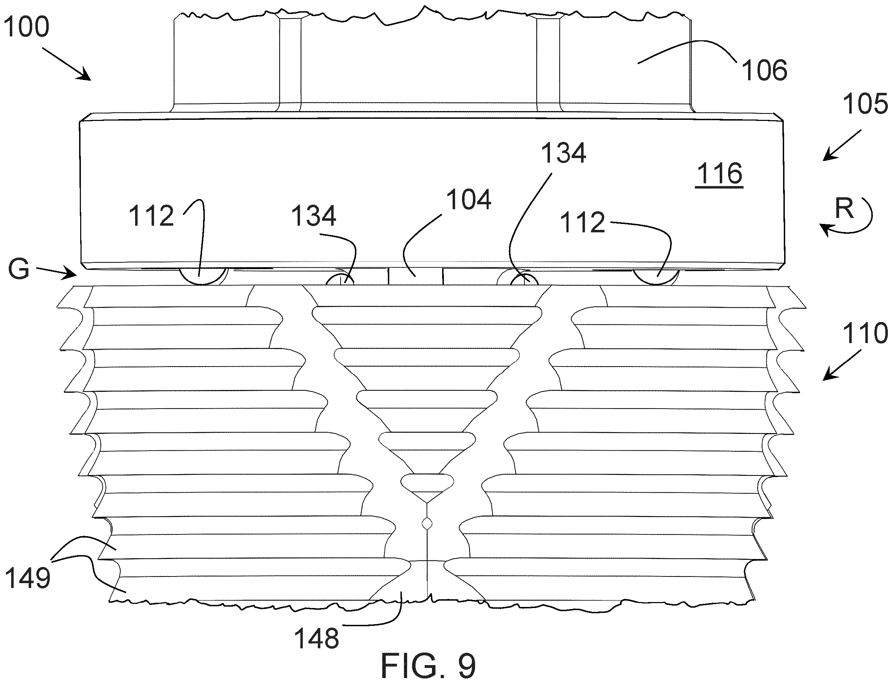

Referring back to **FIGs. 1** and **2****,** the example first medical device **105** (FIG. 1B) and the second example medical device **110** (**FIG. 2**) are shown in a disassembled configuration. Likewise, it will be appreciated that the exemplary medical device connection mechanism **150** (**FIG. 5**) is shown in a disassembled configuration. To place the exemplary orthopedic instrument assembly **100** having an exemplary connection mechanism **150** into an assembled configuration, the interrupted threaded portion **118** of the protrusion **115** can be slid into the threadless channels **123** of the discontinuous threaded portion **121** of the second medical device **110.** Because the threadless channels **123** are desirably keyed slots **123,** it is contemplated that the interrupted threaded portion **118** can be inserted into the threadless channels **123** quickly with limited or no visibility. When the interrupted threaded portion **118** is inserted into the threadless channels **123,** the orthopedic instrument assembly (and by extension, the exemplary medical device connection mechanism **150** itself) **100** can be said to be in a "preassembled" configuration (see **FIGs. 8A** and **9**). Rotating the protrusion **115** and the interrupted threaded portion **118** to engage the threads of the discontinuous threaded portion **121** places the exemplary orthopedic instrument assembly **100** into an "assembled" configuration (see **FIGs. 7** and **8B**).

In the exemplary assemblies depicted in **FIGS. 1** - **8B****,** the protrusion **115** is fixedly engaged to a base **116,** such that the entire first medical device **105** (which is an adaptor in the depicted exemplary embodiments) rotates with the protrusion **115** to place the exemplary orthopedic instrument assembly **100** in the assembled configuration. However, in other exemplary embodiments, it is contemplated that the protrusion **115** can rotate independently of the base **116.**

In embodiments, it is contemplated that markings or other indicia may be provided to indicate the starting position of the preassembled configuration and the engaged position of the fully assembled configuration.

Referring to **FIG. 9****,** which depicts an exemplary orthopedic instrument assembly **100** in a preassembled configuration, a moveable non-ridged spacer **112** is desirably disposed within the gap **G** created between a distal side **103** of the base **116** of the first medical device **105** and the proximal side **124** of the second medical device **110.** In the depicted embodiment, the moveable non-ridged spacer **112** retracts into the base **116** of the first medical device **105** as the orientation of the orthopedic instrument assembly **100** is changed from the preassembled configuration (**FIG. 9**) to the assembled configuration (see **FIG. 7**) via rotation **R.** It will be appreciated that in other exemplary embodiments, the moveable non-ridged spacer **112** can retract into the second medical device **110** as the orientation of the orthopedic instrument assembly **100** is changed from the preassembled configuration to the fully assembled configuration. All types of spacers that can be compressed, deformed, or retracted are considered to be within the scope of this disclosure. Non-limiting examples of moveable non-ridged spacers **112** include a ball plunger, a retractable pin, or an o-ring.

In the embodiments depicted in **FIGS. 1-3****,** two moveable non-ridged spacers **112** are desirably provided at equal and opposite positions within the base **116** to reduce friction evenly as the orientation of the exemplary orthopedic instrument assembly **100** is rotated from the preassembled configuration to the assembled configuration. However, it will be appreciated that in other exemplary embodiments, a single moveable non-ridged spacer **112** may be provided. In yet other exemplary embodiments, more than two moveable non-ridged spacers **112** may be provided. In exemplary embodiments involving two or more moveable non-ridged spacers **112,** it will be appreciated that the two or more moveable non-ridged spacers **112** can be evenly spaced relative to one another, or unevenly spaced.

In the depicted embodiments, a moveable non-ridged spacer receiver **134** is disposed in the proximal surface **122** of the proximal side **124** of second medical device **110.** The moveable non-ridged spacer **112** of the first medical device **105** is disposed within the moveable non-ridged spacer receiver **134** when the orthopedic instrument assembly **100** is in the assembled configuration. It will be appreciated that the moveable non-ridged spacer **112** and the moveable non-ridged spacer receiver **134** (if present) can be disposed on either the first medical device **105,** the second medical device **100,** or both the first medical device **105** and the second medical device **110** in exemplary embodiments.

The depicted first medical device **105** further comprises a pin **104** extending from the distal side **103** of the base **116.** The pin **104** can be used to align the first medical device **105** relative to the second medical device **110** in the preassembled configuration. The user aligns the pin **104** over a first end **166** of an arcuate groove **136** disposed in the proximal side **124** of the second medical device **110** while inserting the protrusion **115** of the first medical device **105** into the threadless channel **123** of the discontinuous threaded portion **121** of the second medical device **110.** The combination of pin **104** and the arcuate groove **136** can provide both quick visual and haptic feedback that the exemplary orthopedic instrument assembly **100** is in the preassembled configuration and is properly aligned to be fully assembled. Stated differently, the pin **104** and the arcuate groove **136** can be desirable user indicators to communicate proper alignment of the timed screw threads **133, 135** of the connection mechanism **150.**

As the user rotates the preassembled orthopedic instrument assembly **100** to place the orthopedic instrument assembly **100** into the assembled configuration, the pin **104** rotates with the base **116** and extends deeper into the opposingly oriented arcuate groove **126** until the pin **104** reaches the accurately distant second end **167** of the arcuate groove **136.** It will be appreciated that the pin **104** and groove **136** (if collectively present) can be disposed on either the first medical device **105,** the second medical device **100,** or both the first medical device **105** and the second medical device **110** in embodiments. It will further be appreciated that the screw threads **133, 135** can be arranged for clockwise or counterclockwise rotation.

In exemplary embodiments comprising timed threads **133, 135,** the gap **G** is effectively eliminated as the users rotates the orthopedic instrument assembly **100** from the preassembled configuration into the assembled configuration, thereby providing further haptic feedback to the user that the first medical device **105** has engaged the second medical device **110.**

Referring collectively to the particular embodiments of **FIGs. 1** - **11****,** these particular examples depict an orthopedic instrument assemblies **100** configured for use in a knee arthroplasty.

Briefly, in a typical knee arthroplasty procedure, the surgeon makes a generally vertical medial parapatellar incision of about five to six inches in length on the anterior or anteromedial aspect of the knee.

The surgeon then continues to incise the fatty tissue to expose the anterior or anteromedial aspect of the joint capsule. The surgeon may then perform a medial parapatellar arthrotomy to pierce the joint capsule. A retractor may then be used to move the patella generally laterally (roughly about 90 degrees) to expose the distal condyles of the femur and the cartilaginous meniscus resting on the proximal tibial plateau. The surgeon then removes the meniscus and uses instrumentation to measure and resect the distal femur and proximal tibia to accommodate trial- and then eventually final - implants.

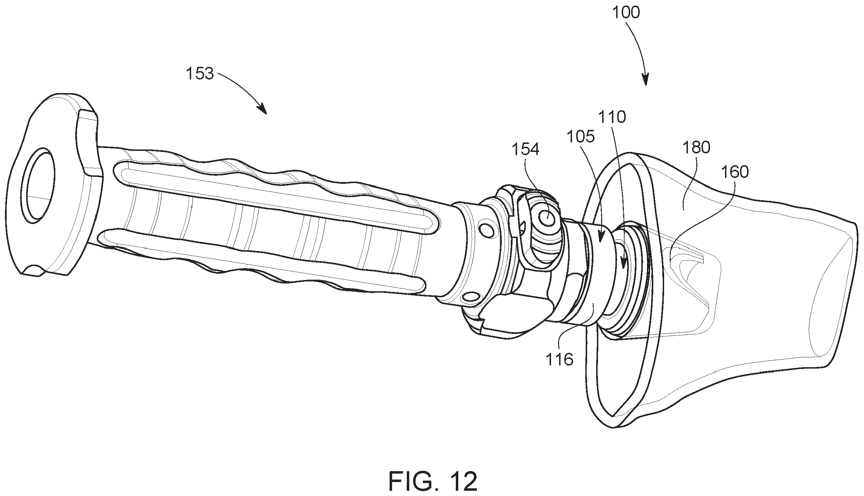

To prepare the resected tibia **180** (**FIG. 12**) for the tibial component of an endoprosthetic knee implant, surgeons can insert a series of progressively larger broaches into the proximal tibial metaphysis and diaphysis to create a pocket in the bone that mirrors the shape and size of the selected implant or implants. Broaching instrumentation often includes a broach (see **FIGS. 2** and **3**) with a mating attachment geometry and an adapter (see **FIG. 1**) that attaches to an impaction handle (see **FIGS. 4** and **5**).

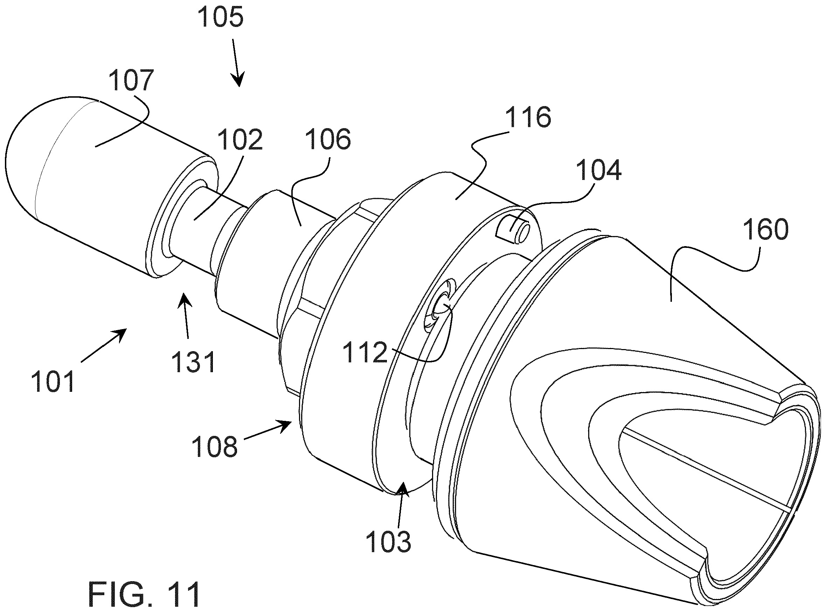

The proximal tibia **180** can present voids and sections of poor bone quality that compromise the overall stability of a tibial implant construct. Surgeons can use conical implants (see **FIGS. 11** and **12**) to fill and reinforce these sections to help prevent further bone degradation and to improve structural integrity of the bone surrounding the tibial implants.

Referring back to **FIG. 1****,** in the depicted example, the first medical device **105** is an adaptor that is configured to mate with the broach of **FIG. 2** (*i.e.*, an example second medical device **110**). The protrusion **115** is a shaft that extends from the base **116** of the adaptor, preferably from the distal side **103** of the base **116.** In preferred embodiments, a pin **104** and a ball plunger (*i.e*., an example moveable non-ridged spacer **112**) also extend from the distal side **103** of the base **116.** Either the pin **104,** or the moveable non-ridged spacer **112,** or both the pin **104** and the moveable non-ridged spacer **112** can be used to help align the timed screw threads **133, 135** and assemble the first medical device **105** and the second medical device **110** in the manner described above.

In the depicted exemplary embodiment, the first medical device **105** (*i.e.*, the adaptor) further comprises a receiver construct **101** extending from a proximal side **108** of the base **116.** The receiver construct **101** comprises a receiver construct base **106** extending from the proximal side **108** of the base **116,** a bridge **102** extending proximally from the receiver construct base **106,** and a receiver backstop **107** extending proximally from the bridge **102.** The bridge **102** desirably has a cross-sectional area that is less than the cross sectional area of the receiver construct base **106** and the receiver backstop **107** when bisected along a transverse plane. In the depicted embodiment, the receiver construct **101** is generally cylindrical, and the bridge **102** and adjacent walls of the receiver construct base **106** and the receiver backstop **107** define an annular gap **131** that can be filled by an engagement protrusion (*e*.*g*., an annular follower **156**) of a positioning instrument (**FIGs. 4** and **5**) to thereby selectively engage the first medical device **105.**

Referring back to **FIGs. 2** and **3****,** in the depicted example, the second medical device **110** is a broach that is configured to mate with the adapter of **FIG. 1****.** The example second medical device **110** comprises an interior sidewall **129.** The interior sidewall **129** defines a hole **127** extending into the body **126** of the second medical device **110.** A discontinuous threaded portion **121** is disposed within the hole **127.** Threads **133** of the discontinuous threaded portion **121** extend from the interior sidewall **129** radially inward toward a center longitudinal axis **C** (**FIG. 8A**) of the hole **127.** The discontinuous threaded portion **121** defines a threadless channel **123** extending along a discontinuous threaded portion height **tpH.** The discontinuous threaded portion **121** can receive the interrupted threaded portion **118** of the protrusion **115** of the first medical device **105** through the threadless channel **123.** The outer side **146** of the depicted second medical device **110** further comprises multiple broach teeth **149** arranged generally circumferentially along the height of the second medical device **110.** The outer side **146** and the multiple broach teeth **149** can define one or more chip breaches **148.** When one of the medical devices in accordance with this disclosure is a broach, chip breaches **148** disposed on the outer side of the broach permit marrow and other metaphyseal and/or diaphyseal tissue to be broken up as "chips" and evacuated from the chip breaches **148** to facilitate broaching. The chip breaches **148** are desirably disposed at an angle on the outer side **146** such that an end of a chip breach **148** located closer to the distal side **147** of the second medical device **110** is offset from an end of the chip breach **148** located closer to the proximal side **124** of the second medical device **110.** Without angled chip breaches **148,** tissue is more likely to be evacuated as longer ribbons, or evacuation could be hindered altogether.

It will be appreciated that the first medical device **105** and the second medical device **110** can be manufactured from materials that have desirable physical and chemical properties for the intended purpose. For example, when the first medical device **105** and the second medical device **110** are reuseable orthopedic instruments, the material may be stainless steel or other suitable clinically tested material. In examples where the first medical device **105,** second medical device **110,** or components thereof are designed to be disposable or for limited use, the materials can be selected from the group consisting essentially of polyether ether ketone ("PEEK"), polyethylene (including but not limited to ultra-high molecular weight polyethylene ("UHMWPE") and crosslinked polyethylene ("XLPE")) ("PE"), and polyamide (including but not limited to a glass-filled polyamide and a carbon fiber filled polyamide).

In exemplary embodiments wherein scratching between the first medical device **105** and the second medical device **110,** or components thereof is desired to be minimized, the first medical device **105,** the second medical device **110,** or engaging components thereof can be manufactured from PEEK. In one exemplary embodiment, the protrusion **118** can be made of PEEK while the remainder of the first medical device **110** is not made from PEEK. In other exemplary embodiments, the first medical device **105,** second medical device **110,** or components thereof can be coated to further reduce visible signs of wear, to further reduce the coefficient of friction, or to accommodate patient allergies. A non-limiting example list of coatings includes cobalt chromium molybdenum alloys, zirconium oxides, and niobium nitrides. In other exemplary embodiments, the threads **135** of the interrupted threaded portion **118** can indirectly engage the threads **133** of the discontinuous threaded portion **121** in the assembled configuration. In such exemplary embodiments, a liner or other intermediate structure can be disposed between the respective threads **133, 135** in the assembled configuration. Such liners can be made from materials selected for the desired purpose. Such materials may include any of the materials listed herein.

**FIGS. 4** and **5** depict an exemplary orthopedic instrument assembly **100** in an assembled configuration that is selectively fixedly engaged to a positioner **153** (*e.g.*, a handle). As better seen in **FIG. 5****,** which is a detailed perspective cross-sectional side view of the embodiment depicted in **FIG. 4****,** the positioner **153** engages the bridge **102** of the receiver construct **101** via a biasing engagement mechanism **155.** All biasing engagement mechanisms **155** known to persons having ordinary skill in the art are considered to be within the scope of this disclosure. In the depicted embodiment, the biasing engagement mechanism **155** comprises a biasing spring **157** disposed within a housing **159** of the positioner **153.** The biasing spring **157** exerts a biasing force to an annular follower **156** that encircles the bridge **102** of the receiver construct **101** of the first medical device **105** when the receiver construct **101** is disposed within the positioner **153.** The annular follower **156** in turn applies the biasing force to the bridge **102,** which distributes these forces through the receiver backstop **107** and the receiver construct base **106** and into the adjacent housing **159** of the positioner **153** to thereby fixedly engage the first medical device **105** of the exemplary orthopedic instrument assembly **100.**

To disengage the first medical device **105** from the positioner **153,** a user can depress the exposed button **154** of the biasing engagement mechanism **155,** which overcomes the biasing force of the biasing spring **157,** thereby releasing the bridge **102** from the annular follower **156** and permitting the user to remove the first medical device **105** from the positioner **153.** In this manner, the exemplary orthopedic instrument assembly **100** can be said to be "selectively fixedly engaged" to a positioner **153.**

**FIG. 5** further depicts an exemplary medical device connection mechanism **150** in the assembled configuration. The threads **135** of the interrupted threaded portion **118** had been inserted through the threadless channel **123** of the of the discontinuous threaded portion **121** of the second medical device **110** (*e.g.*, a broach) and had been rotated to be disposed between and closely received by adjacent threads **133** of the discontinuous threaded portion **121** to thereby engage the threads **133** of the discontinuous threaded portion **121.** To disengage the medical device connection mechanism **150** and thereby place the orthopedic instrument assembly in a disassembled configuration, the user can rotate the second medical device **110** relative to the first medical device **105** such that the threads **135** of interrupted threaded portion **118** disengage the threads **133** of the discontinuous threaded portion **121** and move towards the threadless channel **123** relative to the second medical device **110.** Once the threads **135** of the interrupted threaded portion **118** are in the threadless channel **123,** the first medical device **105** can be withdrawn longitudinally from the second medical device **110** to thereby place the exemplary orthopedic instrument assembly in a disengaged configuration.

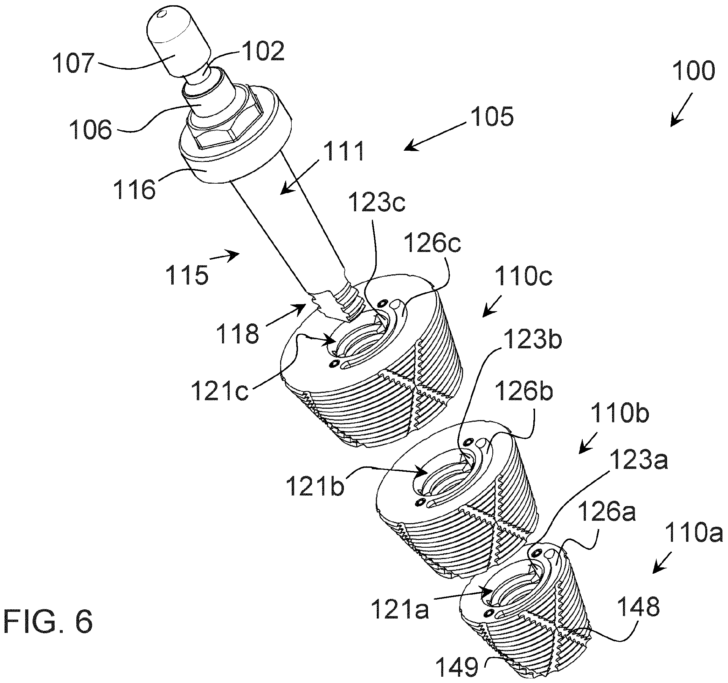

**FIGs. 6** and **7** are perspective views of another exemplary orthopedic instrument assembly **100** comprising an exemplary medical device connection mechanisms **150,** wherein multiple additional medical devices **110b, 110c** are disposed between the first medical device **105** and the second medical device **110a** when the medical device connection mechanism **150** is placed in an assembled configuration. In the depicted embodiment, each additional medical device **110b,110c** disposed between the first medical device **105** and the second medical device **110a** comprise two opposing threadless channels **123b, 123c** and each is capable of engaging an interrupted threaded portion **118** of a protrusion **115** of a first medical device **105** as described above. The first medical device **105** of the depicted embodiment has a longer protrusion **115** than the protrusion **115** depicted in **FIG. 1****.** In practice, the distal end **119** of the protrusion **115** depicted in **FIGs. 6** and **7** can extend through the opposing threadless channels **123c, 123b,** of the additional medical devices **110b, 110c** and into the opposing threadless channels **123a** of the second medical device **110** to place the exemplary orthopedic instrument assembly **100** in a preassembled configuration. When the interrupted threaded portion **118** is rotated out of the opposing threadless channels **123a** of the second medical device **110** to mesh with the adjacent threads **133** of the discontinuous threaded portion **121** of the second medical device **110,** the exemplary orthopedic instrument assembly **100** (and by extension, the exemplary medical device connection mechanism **150**) can be said to be in the "assembled configuration." In **FIGS. 6-7****,** the second medical device **110a** and each additional medical device **110b,110c** (*e.g.*, the third medical device **110b,** and fourth medical device **110c**) are stackable broaches. However, it will be appreciated that other types of medical devices can be used in other embodiments.

In the depicted embodiment, the fourth medical device **110c** is sized to be smaller than the third medical device **110b** to accommodate the natural tapering of the bone into which the assembled orthopedic instrument **100** will in inserted. In exemplary embodiments, the third medical device **110b** can be the same size as the second medical device **100a.** In other exemplary embodiments, the sizes of any of the provided medical devices can differ from the sizes of one or more sizes of the other provided additional medical devices. It is contemplated that additional medical devices **110b, 110c** having exemplary medical device connection mechanisms **150** in accordance with this disclosure, permit true modularity (*i.e.*, the interchangeable use of any of the second, third, fourth, or further medical devices **110a, 110b, 110c...** with the first medical device **105** (*e.g.*, an adaptor)). Without being bound by theory, it is contemplated that exemplary orthopedic instrument assemblies **100** having true modularity as contemplated by this disclosure may permit users to assemble a desired broach assembly quickly to better broach a desired portion of a desired bone in a way that accommodates the patient's unique natural anatomy while minimizing inventory.

It will be appreciated that in other exemplary embodiments, the protrusion **115** can comprise multiple interrupted threaded portions, wherein an interrupted threaded portion of the multiple interrupted threaded portions can further engage a discontinuous threaded portion **121b, 121c** of an additional medical device **110b, 110c** disposed between the first medical device **105** and the second medical device **110** in an assembled configuration. The threads **135, 133** of the respective threaded portions are desirably timed as described above. It will be appreciated that in embodiments comprising multiple additional medical devices **110b, 110c,** the protrusion **115** may be displaced less angularly (when moving from the preassembled configuration to the assembled configuration) compared embodiments in which no additional medical devices **110b, 110c** are present.

**FIG. 8A** is a bottom view of the exemplary orthopedic instrument assembly **100** of **FIGs. 6** and **7** shown in a preassembled configuration. **FIG. 8B** is a bottom view of the exemplary orthopedic instrument assembly **100** of **FIGs. 6** and **7** shown in an assembled configuration.

Although the figures generally depict the hole **127** extending into the center of the body **126** of a second medical device **110** that is generally symmetrical around a bisecting coronal and sagittal plane, nothing in this disclosure limits the invention to the depicted embodiments. In other exemplary embodiments, the hole **127** and therefore the discontinuous threaded portion **121** can be disposed closer to a portion of a distal side **147** of the body **126** of the second medical device **110** than another portion of the distal side **147.** Furthermore, in other exemplary embodiments the first or second medical device **105, 110** can be asymmetric, symmetric around a single bisecting plane, symmetric around multiple bisecting planes, or radially symmetric.

In the embodiments depicted and described with reference to **FIGS. 2** - **8B****,** the second medical device **110** is a bone preparation instrument. Common bone preparation instruments include broaches (see **FIGS. 2** - **8B**), rasps, reamers, keel punches, and other cutting instruments. However, it will be appreciated that in other exemplary embodiments, the second medical device **110** can be an orthopedic instrument other than a bone preparation instrument. Such instruments can include measurement instruments, visualization instruments, and combinations thereof. Combinations and permutations of measurement instruments or visualization instruments with bone preparation instruments are considered to be within the scope of this disclosure.

The example embodiments depicted and described with reference to **FIGS. 2** - 8B, can be particularly adapted to prepare a proximal tibia **180** for one or more conical tibial inserts **160** (see **FIGs. 11** and **12**). Conical tibial inserts **160** are a type of orthopedic endoprosthetic implant. Conical tibial inserts **160** are typically used when an operative tibia **180** presents with voids or sections of poor bone quality. The surgeon can ream, broach, or otherwise remove trabecular bone from the metaphysis and/or diaphysis to create a pocket that generally complements and mirrors the outer dimensions of the conical tibial insert **160.** Once inserted, the conical tibial insert **160** reinforces the metaphyseal and/or diaphyseal sections of the tibia **180** to further reduce bone degradation and to improve the structural integrity of the bone surrounding the tibial components of an endoprosthetic knee implant.

The modular orthopedic instrument assembly **100** of **FIGs. 6** and **7** is an example broaching instrument having an exemplary mechanical connection mechanism **150.** The depicted broaching instrument is desirably also provided with complementary tibial conical inserts **160** that have outer dimensions that mirror or otherwise complement the outer dimensions of the broach construct (*i.e.*, any additional medical devices **110b, 110c** disposed between the first medical device **105** and the second medical device **110**). The tibial conical inserts **160** can desirably be modular tibial conical inserts. In this manner, the surgeon can select sizes and shape of additional medical devices **110b, 110c** (*e.g.*, modular tibial broaches having a component of an exemplary connection mechanism **150** in accordance with this disclosure) that are appropriate for the particular anatomy of the operative patient's tibia **180.** The surgeon can then assemble the exemplary orthopedic instrument assembly **100** in the manner described above. Once assembled, the surgeon can position the broaching instrument over the resected tibia **180** and use a hammer or other impaction tool to broach the proximal tibia **180** to create a pocket that complements the outer dimensions of the provided tibial conical insert(s) **160** (**FIG. 12**).

Because the provided tibial conical insert(s) **160** are complementary to the outer dimensions of the broaches, the created pocket in the tibia **180** can closely receive the tibial conical insert(s) **160.** **FIGs. 10 - 12** illustrate this concept.

**FIG. 10** depicts an exemplary orthopedic instrument assembly **100** in an assembled configuration in which the first medical device **105** is an adaptor and the second medical device **110** is a secondary adaptor configured to selectively engage a conical tibial insert **160** (**FIG. 11**). **FIG. 11** shows the conical tibial insert **160** selectively engaged to the orthopedic instrument assembly **100** via a wedge taper. **FIG. 12** illustrates the exemplary orthopedic instrument assembly **100** of **FIG. 11** further being attached to a positioning member **153** (*e.g.*, a handle), wherein the conical tibial insert **160** is being placed into the pocket in the resected proximal tibia **180** that was created by a broaching orthopedic instrument assembly **100** such as the ones depicted in any of **FIGS. 1 - 8B****.**

In other exemplary embodiments, the second medical device **110** can be an orthopedic implant configured to receive the protrusion **115** of the first medical device **105** (see **FIG. 11**). A non-limiting list of example orthopedic implants include, but is not necessarily limited to: tibial cones, femoral cones, tibial and femoral components of endoprosthetic implants, and trial implants of any of the foregoing.

In other exemplary embodiments, the second medical device **110** can be a secondary adaptor (**FIG. 10**). In such exemplary embodiments, the secondary adaptor can be further configured to selectively engage an orthopedic instrument, or an orthopedic implant, or a further adaptor (see **FIG. 11**). In such exemplary embodiments involving a secondary adaptor, the orthopedic implant, the orthopedic instrument, or further adaptor can be said to be configured to indirectly engage the first medical device **105.**

Other common orthopedic implants include implants or components thereof that extend into the metaphyseal or diaphyseal bone when implanted, other arguments, spacing elements, and void fillers. It will be appreciated that nothing in this disclosure limits the scope of this disclosure to the knee joint. All orthopedic instruments, orthopedic implants, and secondary adaptors having an exemplary connection mechanism **150** are considered to be within the scope of this disclosure.

Components of an exemplary orthopedic instrument assembly **100** can be provided in the form of a surgical kit. The components of the kit are preferably arranged in a convenient format, such as in a surgical tray or case. However, the kit components do not have to be packaged or delivered together, provided that they are assembled or collected together in the operating room for use at the time of surgery.

An exemplary kit can include any suitable embodiment of an exemplary orthopedic instrument assembly **100,** variations of the exemplary orthopedic instrument assemblies **100** described herein, and any other exemplary orthopedic instrument assembly according to an embodiment. While it is contemplated that an exemplary kit may include one or more first medical devices **105** (preferably of difference sizes), one or more second medical devices (preferably of different sizes) and one or more additional medical devices **110b, 110c** (preferably of different sizes) configured to be disposed between the first medical device **105** and the second medical device **110** in the assembled configuration, it will be appreciated that certain kits may lack some or all of these elements.

Any suitable embodiment of a first medical device **105,** variations of the first medical devices **105** described herein, and any other first medical device **105** according to an embodiment, are considered to be within the scope of this disclosure. Any suitable embodiment of a second medical device **110,** variations of second medical devices **110** described herein, and any other second medical device **110** according to an embodiment are considered to be within the scope of this disclosure. Any suitable embodiment of an additional medical device **110b,** variations of the additional medical devices **110b** described herein, and any other additional medical devices **110b** according to an embodiment, are considered to be within the scope of this disclosure.

Selection of a suitable number or type of first medical device **105,** second medical device **110,** and additional medical devices **110b** (**110c**, *etc*.) to include in a kit according to a particular embodiment can be based on various considerations, such as the procedure intended to be performed using the components included in the kit.

An exemplary orthopedic instrument assembly can comprise: an adaptor, the adaptor comprising a shaft having a proximal end and a distal end distally disposed from the proximal end along a height of a body of the shaft, the body of the shaft comprising a shank portion disposed closer to the proximal end than the distal end, and an interrupted threaded portion disposed closer to the distal end than the proximal end; and an orthopedic instrument, the orthopedic instrument having a body, an interior sidewall, the interior sidewall defining a hole extending into the body of the orthopedic instrument, the hole configured to receive the shaft of the adaptor via a discontinuous threaded portion, the discontinuous threaded portion defining a threadless channel extending longitudinally therethrough.

Certain exemplary assemblies can further comprise an engaged configuration, wherein shaft threads of the interrupted threaded portion of the distal end of the shaft adjacently engage threads of the discontinuous threaded form of the orthopedic instrument.

In certain exemplary assemblies, the shaft threads and the discontinuous threaded threads are trapezoidal.

An exemplary orthopedic instrument assembly can comprise: an adaptor, the adaptor comprising a shaft having a proximal end and a distal end distally disposed from the proximal end along a height of a shaft body, the shaft body comprising an interrupted threaded portion disposed closer to the distal end than the proximal end; and an orthopedic instrument, the orthopedic instrument having: a body, an interior sidewall, the interior sidewall defining a hole extending into the body, a discontinuous threaded portion comprising a series of screw threads extending radially inward from the interior sidewall toward a center longitudinal axis, wherein the discontinuous threaded portion defines a threadless channel extending along a height of the discontinuous threaded portion, a threadless channel interior sidewall having: a first threadless channel edge disposed radially outward from and circularly adjacent to a first edge of the interior sidewall to define a first step disposed radially between the first edge of the interior sidewall and the first threadless channel edge, and a second threadless channel edge disposed radially outward from and circularly adjacent to a second edge of the interior sidewall to define a second step disposed radially between the second edge of the interior sidewall and the second threadless channel edge, wherein the screw threads terminate at or before the first step and the second step, wherein the first step, the second step, and the threadless channel interior sidewall define a keyed slot extending longitudinally along the orthopedic instrument, and wherein the keyed slot is configured to closely receive the interrupted threaded portion of the adaptor.

An exemplary orthopedic instrument assembly can comprise: an adaptor, the adaptor comprising a shaft having a proximal end and a distal end distally disposed from the proximal end along a height of a shaft body , the shaft body comprising an interrupted threaded portion disposed closer to the distal end than the proximal end; and an orthopedic instrument, the orthopedic instrument having: a body, an interior sidewall, the interior sidewall defining a hole extending into the body, a discontinuous threaded portion comprising a series of screw threads extending radially inward from the interior sidewall toward a center longitudinal axis, wherein the discontinuous threaded portion defines a threadless channel extending along a height of the discontinuous threaded portion, a first threadless channel interior sidewall having: a first threadless channel edge disposed radially outward from and circularly adjacent to a first edge of the interior sidewall to define a first step disposed radially between the first edge of the interior sidewall and the first threadless channel edge, and a second threadless channel edge disposed radially outward from and circularly adjacent to a second edge of the interior sidewall to define a second step disposed radially between the second edge of the interior sidewall and the second threadless channel edge, wherein the screw threads terminate at or before the first step and the second step, and wherein the first step, the second step, and the threadless channel interior sidewall define a first keyed slot extending longitudinally along the orthopedic instrument, a second threadless channel interior sidewall having: a third threadless channel edge disposed radially outward from and circularly adjacent to a third edge of the interior sidewall to define a third step disposed radially between the third edge of the interior sidewall and the third threadless channel edge, and a fourth threadless channel edge disposed radially outward from and circularly adjacent to a fourth edge of the interior sidewall to define a fourth step disposed radially between the fourth edge of the interior sidewall and the fourth threadless channel edge, wherein the screw threads terminate at or before the third step and the fourth step, wherein the third step, the fourth step, and the second threadless channel interior sidewall define a second keyed slot extending longitudinally along the orthopedic instrument, and wherein the keyed slot is configured to closely receive the interrupted threaded portion of the adaptor.

An exemplary orthopedic medical device connection mechanism comprises: a first medical device having: a protrusion, the protrusion including a proximal end and a distal end distally disposed from the proximal end along a height of a protrusion body, the protrusion body comprising an interrupted threaded portion disposed closer to the distal end than the proximal end; and a second medical device having: a body, an interior sidewall, the interior sidewall defining a hole extending into the body, a discontinuous threaded portion disposed within the hole, the discontinuous threaded portion defining a threadless channel extending along a discontinuous threaded portion height, and the discontinuous threaded portion configured to receive the interrupted threaded portion of the protrusion of the first medical device through the threadless channel.

An exemplary orthopedic medical device connection mechanism can have a protrusion that further comprises a shank portion disposed closer to the proximal end of the protrusion than the distal end.

An exemplary orthopedic medical device connection mechanism can have threads of the interrupted threaded portion and threads of the discontinuous threaded portion having a trapezoidal cross-sectional shape.

An exemplary orthopedic medical device connection mechanism can have the discontinuous threaded portion defining multiple threadless channels extending along the discontinuous threaded portion height. Such an exemplary embodiment may optionally have the discontinuous threaded portion configured to receive the interrupted threaded portion of the protrusion of the first medical device through multiple threadless channels.

An exemplary orthopedic medical device connection mechanism can have threads of the interrupted threaded portion and threads of the discontinuous threaded portion that are timed threads.

An exemplary orthopedic medical device connection mechanism can have the first medical device further comprising a base, wherein the protrusion extends from a distal side of the base, and wherein a moveable non-ridged spacer extends from the distal side of the base. Such an exemplary embodiment may optionally further comprise a pin extending from the distal side of the base. It will be appreciated that such an exemplary medical device connection mechanism may have the second medical device further comprising a proximal side of the second medical device, wherein the proximal side of the second medical device further comprises a moveable non-ridged spacer receiver. In such and exemplary embodiment, the proximal side of the second medical device may optionally further define an arcuate groove configured to closely receive the pin of the first medical device as a configuration of the medical device connection mechanism changes from a preassembled configuration to an assembled configuration.

An exemplary orthopedic medical device connection mechanism can further comprise multiple additional medical devices disposed between the first medical device and the second medical device when the medical device connection mechanism is placed in an assembled configuration.

An exemplary orthopedic medical device connection mechanism can have the first medical device be selected from the group consisting essentially of: an adaptor, a handle, and a positioning member.

An exemplary orthopedic medical device connection mechanism can have the second medical device be selected from the group consisted essentially of: a broach, a reamer, a rasp, a keel punch, and a bone cutting device.

An exemplary orthopedic medical device connection mechanism can have the second medical device be selected from the group consisting essentially of: an endoprosthetic cone, an endoprosthetic implant, and an endoprosthetic implant component.

An exemplary orthopedic medical device connection mechanism can have the second medical device be a secondary adaptor configured to engage an orthopedic instrument, an orthopedic endoprosthetic implant, of a component thereof.

An exemplary orthopedic medical device connection mechanism can have the threadless channel be a keyed slot, the keyed slot being recessed within the body of the second medical device.

An exemplary orthopedic medical device connection mechanism can have the interrupted threaded portion of the protrusion be a keyed interrupted threaded portion.

An exemplary orthopedic medical device connection mechanism comprises: a first medical device having: a protrusion, the protrusion including a proximal end and a distal end distally disposed from the proximal end along a height of a protrusion body, the protrusion body comprising an interrupted threaded portion disposed closer to the distal end than the proximal end; and a second medical device having: a body, an interior sidewall, the interior sidewall defining a hole extending into the body, a discontinuous threaded portion disposed within the hole, the discontinuous threaded portion defining a threadless channel extending along a discontinuous threaded portion height, and the discontinuous threaded portion configured to receive the interrupted threaded portion of the protrusion of the first medical device through the threadless channel, wherein threads of the interrupted threaded portion and threads of the discontinuous threaded portion are timed threads.

## Claims

1. An orthopedic medical device connection mechanism (150) comprising:
a first medical device (105) having:
a protrusion (115), the protrusion (115) including a proximal end (117) and a distal end (119) distally disposed from the proximal end (117) along a height (bH) of a protrusion body (113), the protrusion body (113) comprising an interrupted threaded portion (118) disposed closer to the distal end (119) than the proximal end (117); and
a second medical device (110) having:
a body (126),
an interior sidewall (129), the interior sidewall (129) defining a hole (127) extending into the body (126),
a discontinuous threaded portion (121) disposed within the hole (127), the discontinuous threaded portion (121) defining a threadless channel (123) extending along a discontinuous threaded portion height (tpH), and the discontinuous threaded portion (121) configured to receive the interrupted threaded portion (118) of the protrusion (115) of the first medical device (105) through the threadless channel (123).

2. The orthopedic medical device connection mechanism (150) of claim 1, wherein the protrusion (115) further comprises a shank portion (111) disposed closer to the proximal end (117) of the protrusion (115) than the distal end (119).

3. The orthopedic medical device connection mechanism (150) of claim 1, wherein threads (135) of the interrupted threaded portion (118) and threads (133, 133a, 133b) of the discontinuous threaded portion (121) have a trapezoidal cross-sectional shape.

4. The orthopedic medical device connection mechanism (150) of claim 1, wherein the discontinuous threaded portion (121) defines multiple threadless channels (123) extending along the discontinuous threaded portion height (tpH), and wherein the discontinuous threaded portion (121) is configured to receive the interrupted threaded portion (118) of the protrusion (115) of the first medical device (105) through the multiple threadless channels (123).

5. The orthopedic medical device connection mechanism (150) of claim 1, wherein threads (135) of the interrupted threaded portion (118) and threads (133, 133a, 133b) of the discontinuous threaded portion (121) are timed threads.

6. The orthopedic medical device connection mechanism (150) of claim 1, wherein the first medical device (105) further comprises a base (116), the protrusion (115) extends from a distal side (103) of the base (116), a moveable non-ridged spacer (112) extends from the distal side (103) of the base (116), and a pin (104) extends from the distal side (103) of the base (116).

7. The orthopedic medical device connection mechanism (150) of claim 6, wherein the second medical device (110) further comprises a proximal side (124) of the second medical device (110), wherein the proximal side (124) of the second medical device (110) further comprises a moveable non-ridged spacer receiver (134).

8. The orthopedic medical device connection mechanism (150) of claim 7, wherein the proximal side (124) of the second medical device (110) further defines an arcuate groove (136) configured to closely receive the pin (104) of the first medical device (105) as a configuration of the orthopedic medical device connection mechanism (150) changes from a preassembled configuration to an assembled configuration.

9. The orthopedic medical device connection mechanism (150) of claim 1 further comprising multiple additional medical devices disposed between the first medical device (105) and the second medical device (110) when the orthopedic medical device connection mechanism (150) is placed in an assembled configuration.

10. The orthopedic medical device connection mechanism (150) of claim 1, wherein the first medical device (105) is selected from the group consisting essentially of: an adaptor, a handle, and a positioning member.

11. The orthopedic medical device connection mechanism (150) of claim 1, wherein the second medical device (110) is selected from the group consisted essentially of: a broach, a reamer, a rasp, a keel punch, and a bone cutting device.

12. The orthopedic medical device connection mechanism (150) of claim 1, wherein the second medical device (110) is selected from the group consisting essentially of: an endoprosthetic cone, an endoprosthetic implant, and an endoprosthetic implant component.

13. The orthopedic medical device connection mechanism (150) of claim 1, wherein the second medical device (110) is a secondary adaptor configured to engage an orthopedic instrument, an orthopedic endoprosthetic implant, of a component thereof.

14. The orthopedic medical device connection mechanism (150) of claim 1, wherein the threadless channel (123) is a keyed slot (123a, 123b), the keyed slot (123a, 123b) being recessed within the body (126) of the second medical device (110).

15. The orthopedic medical device connection mechanism (150) of claim 1, wherein the interrupted threaded portion (118) of the protrusion (115) is a keyed interrupted threaded portion (118).
